# EUROPEAN PATENT SPECIFICATION

(11) **EP 4 665 714 B1**
(45) Date of publication and mention of the grant of the patent: **11.11.2026**
(21) Application number: 24708597.0
(22) Date of filing: 23.01.2024
(51) Int. Cl.: C08G 75/02, C08L 65/00, C07C 321/14, C08L 81/02, C09D 165/00, C09D 181/02, C09J 181/02

(54) **POLYTHIOLS, METHODS OF MAKING THEM, AND POLYMERIZABLE COMPOSITIONS**
POLYTHIOLE, VERFAHREN ZU IHRER HERSTELLUNG UND POLYMERISIERBARE ZUSAMMENSETZUNGEN
POLYTHIOLS, LEURS PROCÉDÉS DE FABRICATION ET COMPOSITIONS POLYMÉRISABLES

(30) Priority: 15.02.2023 US 202363485026 P
(43) Date of publication of application: 24.12.2025
(73) Proprietor: PPG Industries Ohio Inc., Cleveland, OH 44111 (US)
(72) Inventor: ZENG, Qi, Murrysville, Pennsylvania 15668 (US); KLEINE, Tristan Stephen, Glenshaw, Pennsylvania 15116 (US)
(74) Representative: f & e patent
(86) International application number: PCT/US2024/012540
(87) International publication number: WO 2024/172999

(56) References cited:
- JP-A- 2022 156 417
- KR-A- 20170 065 273

## Description

### FIELD

The present disclosure is directed to polythiols, methods of making them, and polymerizable compositions that contain them.

### BACKGROUND

Polythiols are used to form optical articles with good thermomechanical properties by reaction with polyisocyanate compounds to form polythiourethane materials. Polythiols with high sulfur content facilitate production of optical articles exhibiting high refractive index. There are many ways to synthesize polythiols, but most techniques involve more than one reaction process step.

Thioacetals have a high sulfur to carbon ratio (yielding a higher refractive index than typical aliphatic polythiols). Additionally, thioacetals may be prepared from aldehydes, which are abundant in the chemical industry and hence inexpensive, are often derived from bio-based sources, are generally benign with respect to environmental and safety concerns, and offer higher selectivity and reactivity (compared to alcohols) for the formation of thioacetals. However, the high reactivity makes unwanted reactions such as oligomerization, polymerization, and crosslinking very likely when a di- or higher thiol is reacted with a di- or higher aldehyde via step growth.

To circumvent this issue, it has been proposed to adopt protection/deprotection reaction schemes, which add steps and cost to the production of the polythiols. Furthermore, mixtures of di-, mono- and unprotected thiols are unavoidable during protection, requiring an intermediate purification step, further adding to the cost and complexity of polythiol synthesis processes. Other approaches exploit the selectivity of thiols over alcohols by reacting mercapto hydroxy compounds with aldehydes, producing polyols as intermediate compounds that may be converted into polythiols by conventional means; again, this adds complexity, time and cost to the synthesis of the polythiols.

There is a need to provide polythiols that may be prepared using minimal reaction process steps without the need for the removal of byproducts or intermediates, and that are suitable for preparing high refractive index polymers.

JP2022156417A discloses polythiols (e.g. TMEE compound) for use in copolymers for lenses.

### SUMMARY

Polythiol compositions are provided, comprising:
a) a polythiol according to Formula (I), 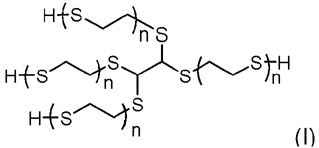 wherein each n is independently from 2 to 4; and
b) a polythiol according to Formula (II) 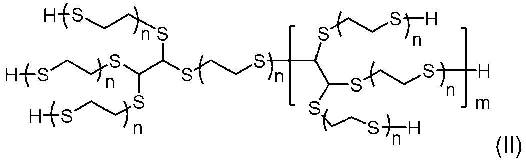 wherein each n is independently from 2 to 4, and m is 1 or 2.

Also provided are methods of preparing the polythiol composition described above, comprising:
a. preparing a reaction mixture comprising a dithiol, a dialdehyde, and a catalyst, wherein the molar ratio of dithiol to dialdehyde is at least 4:1; and
b. subjecting the reaction mixture to conditions sufficient to form the polythiol composition.

Further provided is a polymerizable composition comprising
a) the polythiol composition described above; and
b) an aliphatic polyisocyanate. The equivalent ratio of isocyanate groups (-NCO) in the polyisocyanate b) to thiol (-SH) groups in the polythiol composition a) is from 1:0.9 to 1:1.1.

### DETAILED DESCRIPTION

Notwithstanding that the numerical ranges and parameters setting forth the broad scope of the disclosure are approximations, the numerical values set forth in the specific examples are reported as precisely as possible. Any numerical values, however, inherently contain certain errors necessarily resulting from the standard deviation found in their respective testing measurements.

Any numerical range recited herein is intended to include all sub-ranges subsumed therein. For example, a range of "1 to 10" is intended to include all sub-ranges between and including the recited minimum value of 1 and the recited maximum value of 10, that is, having a minimum value equal to or greater than 1 and a maximum value of equal to or less than 10.

Plural encompasses singular and vice versa; e. g., the singular forms "a," "an," and "the" include plural referents unless expressly and unequivocally limited to one referent. For example, where the composition has been described in terms of "a" polythiol compound or "an" isocyanate, a plurality, including a mixture of such compounds, can be used.

The present disclosure provides polythiols suitable for use in the preparation of optical articles that exhibit high refractive index. It has been found that when an appropriate excess amount of dithiol is reacted with a dialdehyde, a mixture of products is obtained, which is suitable to be used in the formation of optical materials/polythiourethanes, such that the removal of byproducts is unnecessary. The reaction to form the polythiol proceeds in one step, taking place in one pot, without the need for protecting groups nor intermediates that need to be reacted or converted further to the desired polythiol. Consequently, a broad range of compounds containing thioacetal/ketal cores may be easily synthesized using the method described herein.

The polythiol compositions comprise:
a) a polythiol according to Formula (I), 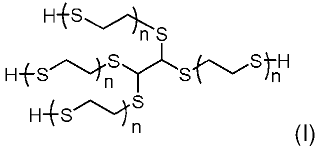 wherein each n is independently from 2 to 4; and
b) a polythiol according to Formula (II) 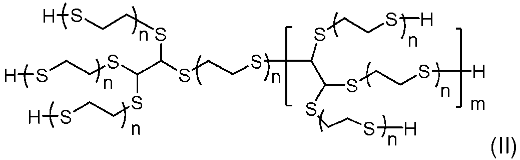 wherein each n is independently from 2 to 4, and m is 1 or 2. Note that each of the components a) and b) may comprise one or more different polythiols having the structures of Formulae (I) and (II).

In certain examples, the polythiol composition may further comprise c) a polythiol according to Formula (III), 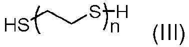 wherein n is from 2 to 4. Often n=2 in Formula (III); however, the polythiol c) may comprise one or more different polythiols having the structure of Formula (III). When the polythiol composition includes the polythiol c) of Formula (III), the polythiol c) may be present in an amount of at least 10 percent, such as at least 20 percent, and at most 50 percent, such as at most 40 percent, based on the total polythiols in the composition as determined by LCMS PROCEDURE defined below. For example, the polythiol according to Formula (III) may be present in the composition in an amount of 10 to 50 percent, or 10 to 40 percent, or 20 to 50 percent, or 20 to 40 percent, based on the total polythiols in the composition as determined by LCMS PROCEDURE defined below. Percent compositions reported from this procedure refer to the area under the curve of a specified compound relative to the sum total of all major UV peaks (>5%) areas under the curve observed in the LC-MS chromatogram. The percentages of each compound are approximately, but not exactly, the same as percent by weight. This is in part because the absorbance of a given compound may not be directly proportional to its weight.

The polythiol composition may be prepared from a reaction mixture comprising a dithiol, a dialdehyde, and a catalyst. Exemplary dithiols have a sufficient chain length (such as greater than 5 atoms) to discourage cyclization. Particularly suitable dithiols include those according to Formula (III), such as 2,2'-thiobis(ethane-1-thiol), or 2,2'-(ethane-1,2-diylbis(sulfanediyl))bis(ethane-1-thiol). Higher polythiols, such as tri-, tetra-, etc., may be included in the reaction mixture as well, but in amounts low enough to minimize formation of higher molecular weight oligomers.

Suitable dialdehydes include low molecular weight, aliphatic dialdehydes, which maximize refractive index in polythiourethane polymerizates and reduce unwanted oligomerization; aromatic dialdehydes may also be used. Difunctional keto carbonyl compounds (aliphatic or aromatic) may also be used but exhibit lower reactivity towards thioketal formation and higher carbon to carbonyl ratios, resulting in decreased refractive index in polythiourethane polymerizates. Poly aldehyde/ketone compounds may also be used but demonstrate a greater propensity to form high molecular weight oligomers. Particular examples of suitable dialdehydes include glyoxal, benzene-1,2-dicarbaldehyde, benzene-1,3-dicarbaldehyde, benzene-1,4-dicarbaldehyde, cyclohexane-1,4-dicarbaldehyde, succinic dialdehyde, or malondialdehyde.

As noted above, when an appropriate excess amount of dithiol is reacted with the dialdehyde, a mixture of products is obtained, but the mixture remains soluble and suitable in the formation of optical materials (i. e., polythiourethanes), such that the removal of byproducts is unnecessary. Typically, the molar ratio of dithiol to dialdehyde in the reaction mixture is at least 4:1, or at least 6:1, such as from 6:1 to 12:1.

Suitable catalysts for use in the reaction mixture include, for example, acid catalysts. Both mineral and organic protic (Brønsted) acids may be used (such as toluene sulfonic acid or hydrochloric acid and the like) at sub-equimolar loadings relative to the dialdehyde. In particular, Lewis acid catalysts such as lithium tetrafluoroborate are suitable; other Lewis acids (such as organometallic compounds, (alkyl)metal chlorides and triflates, organo boranes, etc.) would be suitable as well. The amount of catalyst in the reaction mixture can vary and can be dependent upon a variety of factors such as the type and amounts of the reactive compounds used, as well as reaction conditions, speed of reaction, and degree of reaction desired. Typically, roughly equimolar loadings of Lewis acids relative to the dialdehyde are particularly suitable.

The reaction mixture may further comprise organic solvents, typically polar aprotic solvents as known in the art, which are less likely to react with carbonyl functional groups. Examples include acetonitrile, tetrahydrofuran, dihalomethanes, trihalomethanes, aprotic polyethers, and mixtures thereof . The solvent may be present in amounts of from 25 percent to 95 percent by weight based on the total mass of the reaction mixture.

After the reaction mixture is prepared, it is subjected to conditions sufficient to form the polythiol composition in a one-step, one-pot reaction. The reaction may take place at ambient temperatures. By ambient conditions is meant that the reaction proceeds without the aid of heat or other energy. Usually ambient temperature ranges from 60 to 90 °F (15.6 to 32.2 °C), such as a typical room temperature, 72°F (22.2°C). Reaction conditions may include heating to a temperature of from 30 to 85°C, such as 40 to 75°C, or 55 to 65°C, for 1 to 24 hours, such as 10 to 15 hours. Exemplary reaction conditions are 58 to 62°C for 11 to 13 hours. Water liberated from the carbonyl species may be actively scavenged and removed from the reaction by addition of desiccants or distillation, but is typically not necessary for the reaction to proceed.

The polythiol composition may have a thiol equivalent weight of at least 100 g/equivalent, or at least 110 g/equivalent, or at least 120 g/equivalent, and at most 250 g/equivalent, such as at most 220 g/equivalent, or at most 200 g/equivalent, or at most 160 g/equivalent, based on the total mass of polythiols present in the composition. For example, the polythiol composition may have a thiol equivalent weight of 100 to 250 g/equivalent, or 100 to 220 g/equivalent, or 100 to 200 g/equivalent, or 100 to 160 g/equivalent, or 110 to 250 g/equivalent, or 110 to 220 g/equivalent, or 110 to 200 g/equivalent, or 110 to 160 g/equivalent, or 120 to 250 g/equivalent, or 120 to 220 g/equivalent, or 120 to 200 g/equivalent, or 120 to 160 g/equivalent, based on the total mass of polythiols present in the composition.

Typically, the polythiol of Formula (I) may be present in the polythiol composition in an amount greater than 20 percent, such as at least 25 percent, or at least 30 percent, and at most 50 percent, or at most 45 percent, based on the total polythiols in the composition as determined by LCMS PROCEDURE defined below. For example, the polythiol of Formula (I) may present in the polythiol composition in an amount of 20 to 50 percent, or 20 to 45 percent, or 25 to 50 percent, or 25 to 45 percent, or 30 to 50 percent, or 30 to 45 percent, based on the total polythiols in the composition as determined by LCMS PROCEDURE defined below.

The use of the above-noted reactants in their given ratios allows for the formation of polythiols having thioacetal "cores", which provide the advantages discussed above to a polythiourethane polymerizate. The polythiol compositions described above are thus useful in polymerizable compositions to form optical polymerizates with refractive indices (nₑ²⁰) greater than or equal to 1.60. Such polymerizable compositions comprise: a) a polythiol composition as described above; and b) an aliphatic polyisocyanate.

The polyisocyanate is aliphatic, but may further include aromatic polyisocyanates. Isocyanate functional uretdiones, allophanates, biurets, and isocyanurates are also suitable. Diisocyanates and triisocyanates such as isocyanurates of diisocyanates are often used. Isocyanate-functional prepolymers, for example reaction products of polyisocyanates with polyols also can be used. Mixtures of polyisocyanates can be used.

The polyisocyanate can be prepared from a variety of isocyanate-containing materials. Other examples of suitable polyisocyanates include trimers prepared from the following diisocyanates: 4,4'-methylene-bis(cyclohexyl isocyanate), isophorone diisocyanate, an isomeric mixture of 2,2,4- and 2,4,4-trimethyl hexamethylene diisocyanate, 1,6-hexamethylene diisocyanate, and 4,4'-diphenylmethylene diisocyanate.

Suitable diisocyanates include, 4,4'-methylene-bis(cyclohexyl isocyanate) (i. e., 4,4'-diisocyanato dicyclohexylmethane), isophorone diisocyanate, an isomeric mixture of 2,2,4- and 2,4,4-trimethyl hexamethylene diisocyanate, and 1,6-hexamethylene diisocyanate. Usually the equivalent ratio of isocyanate groups (-NCO) in the polyisocyanate b) to thiol (-SH) groups in the polythiol composition a) is from 1:0.9 to 1:1.1, often 1:1.

Examples of aromatic polyisocyanates that may be used in addition to the aliphatic polyisocyanate include: toluene diisocyanate, tetramethyl xylylene diisocyanate, m-xylenediisocyanate. Uretdiones, allophanates, biurets, isocyanurates, and isocyanate-functional prepolymers prepared therefrom are also suitable.

The polythiol composition may further comprise urethanation catalysts, color correcting dyes, mold release agents, degassing agents, UV absorbers, and the like, as known in the art, present in art-recognized amounts. The amounts of each additive will vary depending on the desired performance and intended use of the article.

Suitable urethanation catalysts can vary; for example, suitable urethanation catalysts can include those catalysts that are useful for the formation of thiourethane by reaction of the NCO and SH-containing materials. Non-limiting examples of suitable catalysts can be chosen from the group of Lewis bases, Lewis acids and insertion catalysts as described in Ullmann's Encyclopedia of Industrial Chemistry, 5th Edition, 1992, Volume A21, pp. 673 to 674. The catalyst can be a stannous salt of an organic acid, such as but not limited to stannous octoate, dibutyl tin dilaurate, dibutyl tin diacetate, dibutyl tin mercaptide, dibutyl tin dimaleate, dimethyl tin diacetate, dimethyl tin dilaurate, dibutyltin dichloride, dimethyl tin dichloride, 1,4-diazabicyclo[2.2.2]octane, and mixtures thereof. The catalyst can alternately be zinc octoate, bismuth, or ferric acetylacetonate.

Further non-limiting examples of suitable catalysts can include tin compounds such as such as dibutyl tin oxide, phosphines, tertiary ammonium salts and tertiary amines such as but not limited to triethylamine, triisopropylamine, dimethyl cyclohexylamine, N,N-dimethylbenzylamine, pyridine, and mixtures thereof. Amine compounds and alkyl tin halide compounds are most commonly used and are most suitable when used in conjunction. Catalyst levels can vary depending on the type used; for example, an organotin catalyst is typically present in an amount up to 1000 ppm, such as 100 to 1000 ppm; often about 250 ppm. Mixtures two or more of the catalysts above are also suitable.

Polymerization of the composition described above results in the formation of a polymerizate, which can be made in the form of a shaped article, such as by casting to form a sheet or by molding. Polymerizates obtained from polymerization of the polymerizable compositions of the present disclosure are thermoset solids, and in some embodiments, transparent.

The polymerizable compositions may alternatively be sprayable, castable, extrudable, 3-D printable, or moldable. Polymerizates prepared from the polymerizable compositions are often used to form solid articles such as optical element(s) or device(s). As used herein the term "optical" means pertaining to or associated with light and/or vision.

In the making of an optical article, the polymerizable composition may be introduced into a mold of any desired shape at a temperature and for a time to form a polymerizate. After mixing of the components of the polymerizable composition to form a reaction mixture it is introduced, usually by injection, into a mold. The mold may have any shape desired for the final product as noted above. It is typically a lens mold; often a mold for an ophthalmic lens. The molded article may then be released from the mold.

The optical article can comprise ophthalmic elements and devices, and sheet products such as display elements and devices, windows, mirrors, and/or active and passive liquid crystal cell elements and devices. As used herein the term "ophthalmic" means pertaining to or associated with the eye and vision. Non-limiting examples of ophthalmic elements include corrective and non-corrective (plano) lenses, including single vision or multi-vision lenses, which may be either segmented or non-segmented multi-vision lenses (such as, but not limited to, bifocal lenses, trifocal lenses and progressive lenses), as well as other elements used to correct, protect, or enhance (cosmetically or otherwise) vision, including without limitation, contact lenses, intraocular lenses, magnifying lenses, sun lenses, fashion lenses, sport masks, face shields and goggles. As used herein the term "display" means the visible or machine-readable representation of information in words, numbers, symbols, designs or drawings. Non-limiting examples of display elements and devices include screens, including touch screens, monitors, and security elements, such as security marks. As used herein the term "window" means an aperture adapted to permit the transmission of radiation there-through. Non-limiting examples of windows include automotive and aircraft transparencies, filters, shutters, and optical switches. As used herein the term "mirror" means a surface that specularly reflects incident light.

Polymerizates prepared from the polymerizable compositions may have: a refractive index of at least 1.57, or at least 1.58, or at least 1.59; an ABBE number of at least 30, or at least 33, or at least 35; and a Fischer microhardness value of at least 50 N/mm², or at least 70 N/mm², or at least 90N/mm². The refractive index, ABBE number, and Fischer Hardness values can be determined in accordance with art-recognized methods; for example, refractive index values (nₑ²⁰) and ABBE numbers may be determined using a Metricon Model 2010 Prism Coupler, Thin Film Thickness / Refractive Index Measurement System, in accordance with the manufacturer's Operation and Maintenance Guide; and Fischer Hardness values are determined in accordance with ISO 14577 using a Fischer Technologies H100C Microhardness Measurement System.

The following working Examples are intended to further describe the disclosed compositions. It is understood that the disclosure in this specification is not necessarily limited to the examples described in this section. Components that are mentioned elsewhere in the specification as suitable alternative materials for use in the compositions, but which are not demonstrated in the working Examples below, are expected to provide results comparable to their demonstrated counterparts. Unless otherwise indicated, all parts are by weight.

### EXAMPLES

### Definitions/Methods:

### Part 1.

### Example 1: Preparation of polythiol composition

Glyoxal (64.90 g of a 40 wt/wt% solution in water, 0.45 mol), 2,2'-thiobis(ethane-1-thiol) (482.8 g, 3.13 mol), and lithium tetrafluoroborate (75.4 g, 0.80 mol) were charged sequentially to acetonitrile (1621 mL) and dissolved to form a solution in a 5L four-necked, round-bottomed flask. The reaction solution was heated to 60 °C for 12 hours and then cooled to room temperature, whereupon the mixture phase separated, and the bottom layer containing the product was collected. This product layer was washed with two portions of acetonitrile sequentially, first 300 mL and then 150 mL; the acetonitrile washing layers were discarded. The remaining acetonitrile insoluble fraction was concentrated under vacuum at 50-60 °C to form a clear, viscous liquid product (206 g) that was passed through a 5 µm filter. Prior to use, the polythiol mixture was dried under high vacuum for a minimum of 8 hours to remove residual moisture.

### Part 2. Characterization of polythiol composition of Example 1.

Thiol equivalent weights were determined using the following procedure. A sample (0.0500 - 0.1000 g) of polythiol was weighed on an analytical balance to the ten-thousandths place and dissolved with 30 mL of either tetrahydrofuran or a 60/40 wt/wt solution of toluene/isopropanol, depending on solubility of the polythiol. 1-3 drops of pyridine were added to the solution, which was then stirred at room temperature until the sample was dissolved. With stirring, a Metrohm 865 Dosimat Plus was used to titrate in a 0.1 N solution of iodine in water, until a distinct yellow color persisted. This process was repeated and the SH equivalent weight was calculated using the Equation (3) below and an average of the two results used for calculations. It is acknowledged that certain polythiol mixtures used in the examples/comparative examples may contain a small amount of residual hydroxyl functional groups; potentially affecting the total active hydrogen equivalent weights and thus the ratio of active hydrogen groups relative to isocyanate groups. However, this difference is expected to be negligible and thiol equivalent weights alone have been used to calculate the required amount of isocyanate groups to reach approximately a 1:1 ratio of active hydrogen groups : isocyanate groups. Equation (3): $SH \left(\frac{g}{eq}\right) = \frac{mass analyte \left(g\right) \times 1000}{volume titrant \left(mL\right) \times N factor of titrant}$

The thiol equivalent weight of Example 1 was determined according the procedure described and found to be 224 g/eq.

The product was also characterized by liquid chromatography coupled with mass spectrometry according to the following procedure ("LCMS PROCEDURE"), to determine the amounts of each polythiol component in the product mixture. The product mixture was diluted with tetrahydrofuran (THF) and analyzed by reverse phase ultra-high pressure liquid chromatography with online UV/Vis and mass spectrometry detection. The mixture of products was separated on a Dionex UHPLC with a Waters Cortecs UPLC C18 column with a 1.6 µm pore size and dimensions 100 x 2.1 mm. The mobile phase was a gradient mixture of deionized water and acetonitrile ranging from 60% DI H2O / 40% acetonitrile at T0 and ramping up to 2% DI H2O / 98% acetonitrile by the end of the run. Analytes were detected using a Vanquish UV-Vis set at 230 nm. Mass spectrometry was performed on the UPLC effluent in both full MS with AIF ESI positive mode and Full MS with AIF ESI negative mode in the scan range of 133.4 - 2000 m/z on a QExactive Mass Spectrometer. Data analysis was performed using Xcalibur 4.2 software. Again, percent compositions reported from this analysis refer to the area under the curve of a specified compound, relative to the sum total of all major UV peaks (>5%) areas under the curve observed in the LC-MS chromatogram.

The composition according to this analysis is shown in Table 1.

**Table 1: polythiol composition breakdown of Example 1.**

| Compound | Retention Time (min) | % Area | Observed mass (g/mol) | Proposed molecular formula | Exact Mass (g/mol) |
|---|---|---|---|---|---|
| Formula (I) (n = 2) | 10.71 | 41.2 | 655.9964 | *C₁₈H₄₂NS₁₂⁺ | 655.9960 |
| Formula (II) (m = 1; n = 2) | 13.92 | 15.4 | 1139.9646 | *C₃₂H₇₀NS₂₁⁺ | 1139.9638 |
| Formula (II) (m = 2; n = 2) | 15.45 | 4.3 | 1623.9333 | *C₄₆H₉₈NS₃₀⁺ | 1623.9315 |
| Formula (III) (n = 2) | 2.47 | 19.7 | 196.0284 | *C₄H₁₀S₃+C₂H₃N+H⁺ | 196.0283 |

| | | | | | |
|---|---|---|---|---|---|
| *complexes of the molecular ion and supporting electrolyte | | | | | |

### Part 3. Preparation of curable compositions.

**Table 2: Composition of examples/comparative examples**

| Charge | Component | Parts by weight | | | | |
|---|---|---|---|---|---|---|
| | | CE-2 | CE-3 | 4 | 5 | 6 |
| 1 | 4,4'-diisocyanato dicyclohexylmethane | 19.7 | - | - | 19.78 | 29.01 |
| | 1,3-Bis(isocyanatomethyl) cyclohexane | 6.6 | - | - | 6.60 | 9.67 |
| | Isophoronediisocyanate | - | - | 20.05 | - | - |
| | meta-xylene diisocyanate | - | 22.23 | - | - | - |
| 2 | Dimethyltin dichloride | 0.0142 | 0.0088 | 0.0156 | 0.0205 | 0.0204 |
| 3 | TRIBRID^{®} Component E¹ | 0.0667 | | 0.24 | 0.105 | 0.0938 |
| | ZELEC^{®} UN² | | 0.0888 | | | |
| 4 | Polythiol of Example 1 | - | 53.03 | 40.12 | 49.3 | 16.53 |
| | HIRI^{™} 167 Lens Material Component B1³ | 2.35 | - | - | - | - |
| | Pentaerythritol tetrakis(2-mercaptoacetate)⁴ | 17.64 | - | - | - | - |
| | Polythiol 2⁵ | 1.17 | - | - | - | - |
| | 2,2'-thiobis(ethane-1-thiol) | 1.17 | - | - | - | 20.21 |
| NCO eq. wt. of Charge 1⁶ | | 120.6 | 94.09 | 111.2 | 120.6 | 120.6 |
| SH eq. wt. of Charge 4⁷ | | 108.3 | 224.0 | 224.0 | 224.0 | 110.3 |

| | | | | | | |
|---|---|---|---|---|---|---|
| ¹ A blend of internal mold release agents containing 13.8% triethylamine, based on GC-FID analysis available from PPG Industries, Inc. ² A mold release agent available from Stepan Company ³ A mixture of primarily 2,3-bis((2-mercaptoethyl)thio)-1-propanethiol and associated isomers, available from PPG Industries, Inc. ⁴ Obtained from TCI AMERICA at >90% purity ⁵ The 2/1 (mol/mol) adduct of 2,2'-thiobis(ethane-1-thiol) and propargyl alcohol previously described in US 7,687,597 B2. ⁶ Equivalent weights of mixtures were determined by titration. ⁷ Determined by titration as described above. | | | | | | |

### Comparative Example CE-2: Optical articles cast from cycloaliphatic diisocyanates and industry standard polythiols

The components of Charge 1 were added to a 250 mL flask with a vacuum adapter and equipped with a magnetic stir bar. To this was added Charge 2 and Charge 3. This mixture was placed under vacuum and degassed for approximately 30 minutes. The thiol mixture of Charge 4, calculated to achieve a thiol : isocyanate equivalent ratio of 1:1, was charged to the reaction flask which was sealed and placed under vacuum again. The mixture was stirred at room temperature for approximately 2 hours, after which point the temperature was raised to 60 °C by placing the flask in an oil bath. After 5 minutes, the mixture was poured into a pre-warmed (60 °C) flat glass mold with rubber gasket spacers with thickness approximately 3.5 mm. The mold was placed in an oven initially set at 60 °C. The oven temperature was raised to 140 °C over the course of 9.16 hours, held at this temperature for 5.16 hours and finally ramped down to 70 °C over the course of 1.16 hours then the oven was turned off and sample allowed to return to room temperature where it was removed from the mold This same procedure and ratios of reagents was used to prepare another polymerizate mixture; this mixture was cast into a pre-assembled, finished single vision minus "-" powered mold with set center thickness of 2.2 mm. The same curing conditions were also employed.

### Comparative example CE-3: Optical articles cast from aromatic diisocyanate and polythiol of Example 1

Charge 1 was added to a 250 mL flask with a vacuum adapter and equipped with a magnetic stir bar. To the flask was also added Charge 2 and Charge 3. This mixture was placed under vacuum and degassed for approximately 35 minutes. The polythiol of Charge 4, calculated to achieve a thiol : isocyanate equivalent ratio of 1:1, was charged to the reaction flask and placed back under vacuum. The mixture was immediately homogeneous and stirred for approximately 15 minutes at room temperature before being dispensed into a pre-assembled finished single vision minus "-" powered mold with set center thickness of 2.2 mm. The mold was placed in an oven initially set at 60 °C. The oven temperature was raised to 140 °C over the course of 9.16 hours, held at this temperature for 5.16 hours and finally ramped down to 70 °C over the course of 1.16 hours then the oven was turned off and sample allowed to return to room temperature where it was removed from the mold.

### Example 4: optical flat sheet cast from IPDI and polythiol of Example 1

Charge 1 was added to a 100 mL round bottomed flask equipped with a magnetic stir bar and degassed under high vacuum for approximately 20 minutes. The components of Charges 2 and 3 were added, the flask was resealed and degassed again. The polythiol mixture of Charge 4 was then added to the flask, calculated to achieve a thiol : isocyanate equivalent ratio of 1:0.99. The flask was sealed once more and placed under high vacuum. The reaction mixture was stirred at 60 °C in an oil bath until it was homogenous, c.a. 30 minutes. The mixture was aged for approximately 20 minutes further at 60 °C then poured into a flat glass mold with rubber gasket spacers at a thickness of approximately 3.5 mm. The polymerizate in the mold was transferred to an oven initially at ambient conditions. The oven temperature was raised to 140 °C over the course of 9.75 hours, held at this temperature for 5.16 hours and finally ramped down to 70 °C over the course of 1.16 hours. The oven was turned off and samples allowed to return to room temperature where they were removed from their molds.

### Example 5: Optical articles cast from cycloaliphatic diisocyanates and polythiol of claim 1

The components of Charge 1 were added to a 250 mL flask with a vacuum adapter and equipped with a magnetic stir bar; to the same flask was also added Charge 2 and Charge 3. This mixture was placed under vacuum and degassed for approximately 20 minutes. The polythiol of Charge 4, calculated to achieve a thiol : isocyanate equivalent ratio of 1:1.01, was charged to the reaction flask and placed back under vacuum. The mixture under vacuum was placed in a 60 °C oil bath and aged for 60 minutes, during which the mixture became homogenous. The reaction mixture was dispensed into a pre-assembled finished single vision minus "-" powered mold with set center thickness of 2.2 mm and a flat glass mold with rubber gasket spacers of thickness approximately equal to 3.5 mm. The molds were placed in an oven initially set at 60 °C. The oven temperature was raised to 140 °C over the course of 9.16 hours, held at this temperature for 5.16 hours and finally ramped down to 70 °C over the course of 1.16 hours then the oven was turned off and samples allowed to return to room temperature where they were removed from their molds.

### Example 6: Optical articles cast from cycloaliphatic diisocyanates and polythiol from claim 1 with lower SH equivalent weight

The components of Charge 1 were added to a 250 mL flask with a vacuum adapter and equipped with a magnetic stir bar. To the same flask was added Charge 2 and Charge 3. This mixture was placed under vacuum and degassed for approximately 50 minutes. The polythiol mixture of Charge 4, calculated to achieve a thiol : isocyanate equivalent ratio of 1:1.04 was charged to the reaction flask and placed back under vacuum. The mixture was placed in a 60 °C oil bath and aged for approximately 15 minutes whereupon the mixture became homogenous. After aging for a further 30 minutes approximately, the reaction mixture was dispensed into a pre-assembled, finished single vision minus "-" powered mold with set center thickness of 2.2 mm and a flat glass mold with rubber gasket spacers of thickness approximately equal to 3.5 mm. The molds were placed in an oven initially set at 60 °C. The oven temperature was raised to 140 °C over the course of 9.16 hours, held at this temperature for 5.16 hours and finally ramped down to 70 °C over the course of 1.16 hours then the oven was turned off and samples allowed to return to room temperature where they were removed from their molds.

### Part 4. Evaluation of polymerizates of Examples and Comparative Examples.

Fischer microhardness (FMH) was determined by testing according to ISO 14577-07 using a FISHERSCOPE^{®} H-100SMC (available from Fisher Technology, Inc.). The FMH of polymerizates was measured at a load of 300 mN, following a load application of 0-300 mN in 15 seconds. The results are an arithmetic average of at least 3 measurements.

Glass transition temperature was measured on a Q200 differential scanning calorimeter (DSC) from TA Instruments.

Refractive index was measured using a Metricon Model 2010/M Prism Coupler equipped with monochromatic light sources at 453, 543 and 633 nm and corrected for temperature. Abbe number was calculated from the refractive index values according to the below equations (1) and (2) where V_{D} and Vₑ are the Abbe values with respect to the Fraunhofer d- and green mercury E-lines respectively, and n_{d}, n_{F}, n_{C}, nₑ, n_{F'} and n_{C'} are the refractive indices of the material at the Fraunhofer D-, F-, C- lines, green mercury E-line, blue and red cadmium lines, respectively. Refractive indices at wavelengths not directly measured were calculated from a best fit of the empirical data to Cauchy's equation. Equation (1): ${V}_{D} = \frac{{n}_{d} - 1}{{n}_{F} - {n}_{C}}$ Equation (2): ${V}_{e} = \frac{{n}_{e} - 1}{{n}_{F ′} - {n}_{c ′}}$

Luminous transmittance and color were measured using a HunterLab Ultra Scan Pro spectrophotometer (available from Hunter Associates Laboratory, Inc.) according to ASTM E313-10°/D65 and the manufacturer's instructions. The path length for sheet samples is equal to the sample thickness.

**Table 3: Physical/optical properties of examples 4-6 and comparative examples CE-2 and CE-3; data represented herein obtained from flat sheets**

| Entry | FMH (N/mm²) | T_{g} (°C) | n_{d} | V_{d} | Thickness (mm) |
|---|---|---|---|---|---|
| Comparative Example CE-2 | 170 | 114 | 1.57 | 44 | 3.19 |
| Comparative Example CE-3 | 10 | 72 | 1.68 | 32 | 3.32 |
| Example 4 | 147 | 60 | 1.64 | 37 | 3.32 |
| Example 5 | 126 | 53 | 1.64 | 37 | 3.20 |
| Example 6 | 134 | 72 | 1.61 | 39 | 3.26 |

| Entry | luminous transmittance (%) | L* | a* | b* |
|---|---|---|---|---|
| Comparative Example CE-2 | 90.6 | 96.23 | -0.07 | 0.37 |
| Comparative Example CE-3 | 54.1 | 78.52 | -0.20 | 16.73 |
| Example 4 | 85.2 | 93.98 | -1.73 | 8.25 |
| Example 5 | 85.8 | 94.24 | -1.03 | 5.50 |
| Example 6 | 89.0 | 95.60 | -0.37 | 1.82 |

As demonstrated above, Examples 4, 5 and 6 demonstrate high refractive indexes as compared to CE-2 with lower thiol equivalent weight components. In addition, the aromatic polyisocyanate of CE-3 forms a very soft polymerizate with poor luminous transmittance. Specifically, the examples above demonstrate that that high refractive index can be achieved with acceptable thermomechanical properties.

## Claims

1. A polythiol composition comprising:
a) a polythiol according to Formula (I) 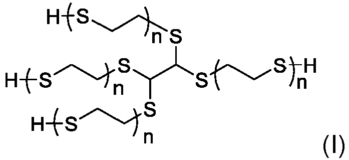 wherein each n is independently from 2 to 4; and
b) a polythiol according to Formula (II) 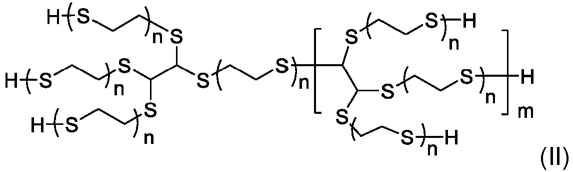 wherein each n is independently from 2 to 4, and m is 1 or 2.

2. The polythiol composition of claim 1, wherein the polythiol composition has a thiol equivalent weight of from 100 to 250 g/equivalent, preferably of from 120 to 160 g/equivalent, based on the total mass of polythiols present in the composition.

3. The polythiol composition of any of the preceding claims, further comprising c) a polythiol according to Formula (III), 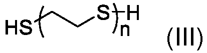 wherein n is from 2 to 4.

4. The polythiol composition of claim 3, wherein n = 2 in Formula (III).

5. The polythiol composition of claim 4, wherein the polythiol according to Formula (III) is present in the composition in an amount of 10 to 50 percent, based on the total polythiols in the composition as determined by LCMS PROCEDURE.

6. The polythiol composition of any of the preceding claims, wherein the polythiol composition is prepared from a reaction mixture comprising a dithiol, a dialdehyde, and catalyst, and wherein the molar ratio of dithiol to dialdehyde is at least 4:1, preferably from 6:1 to 12:1.

7. The polythiol composition of claim 6, wherein the dithiol comprises the polythiol according to Formula (III) wherein n=2; and/or
wherein the dialdehyde comprises glyoxal; and/or
wherein the catalyst comprises lithium tetrafluoroborate.

8. A method of preparing the polythiol composition of any of the preceding claims, comprising:
a. preparing a reaction mixture comprising a dithiol, a dialdehyde, and a catalyst, wherein the molar ratio of dithiol to dialdehyde is at least 4:1; and
b. subjecting the reaction mixture to conditions sufficient to form the polythiol composition.

9. The method of claim 8, wherein the molar ratio of dithiol to dialdehyde is at least 6:1, preferably from 6:1 to 12:1.

10. The method of any of claims 8 to 9, wherein the dithiol comprises
a polythiol according to Formula (III), 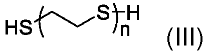
wherein n is from 2 to 4.

11. The method of any of claims 8 to 10, wherein the dialdehyde comprises glyoxal; and/or
wherein the catalyst comprises lithium tetrafluoroborate.

12. The method of any of claims 8 to 11, wherein the conditions sufficient to form the polythiol composition comprise heating to a temperature of from 30 to 85°C for 1 to 24 hours.

13. A polymerizable composition comprising
a) the polythiol composition of any of claims 1 to 7; and
b) an aliphatic polyisocyanate, wherein the equivalent ratio of isocyanate groups (-NCO) in the polyisocyanate b) to thiol (-SH) groups in the polythiol composition a) is from 1:0.9 to 1:1.1.

14. The polymerizable composition of claim 13, wherein the polyisocyanate b) comprises 4,4'-methylene-bis(cyclohexyl isocyanate), isophorone diisocyanate, an isomeric mixture of 2,2,4- and 2,4,4-trimethyl hexamethylene diisocyanate, and 1,6-hexamethylene diisocyanate, or mixtures thereof.

15. An optical article formed from the polymerizable composition of any of claims 13 to 14.

## Patentansprüche

1. Eine Polythiolzusammensetzung umfassend:
a) ein Polythiol gemäß Formel (I) 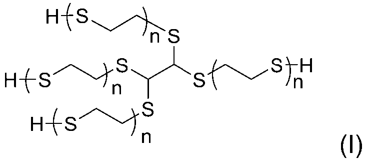 wobei jedes n unabhängig von 2 bis 4 beträgt; und
b) ein Polythiol gemäß der Formel (II) 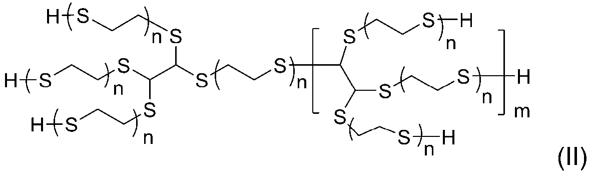 wobei jedes n unabhängig von 2 bis 4 beträgt und m 1 oder 2 beträgt.

2. Die Polythiolzusammensetzung gemäß Anspruch 1, wobei die Polythiolzusammensetzung ein Thioläquivalentgewicht von 100 bis 250 g/Äquivalent aufweist, vorzugsweise von 120 bis 160 g/Äquivalent, bezogen auf das Gesamtgewicht von Polythiolen, die in der Zusammensetzung vorhanden sind.

3. Die Polythiolzusammensetzung gemäß irgendeinem der vorhergehenden Ansprüche, des Weiteren umfassend:
c) ein Polythiol gemäß der Formel (III) 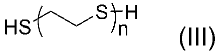 wobei n von 2 bis 4 beträgt.

4. Die Polythiolzusammensetzung gemäß Anspruch 3, wobei n = 2 in Formel (III).

5. Die Polythiolzusammensetzung gemäß Anspruch 4, wobei das Polythiol gemäß Formel (III) in der Zusammensetzung in einer Menge von 10 bis 50%, bezogen auf alle Polythiole in der Zusammensetzung, wie durch das LCMS-VERFAHREN bestimmt, vorhanden ist.

6. Die Polythiolzusammensetzung gemäß irgendeinem der vorhergehenden Ansprüche, wobei die Polythiolzusammensetzung aus einer Reaktionsmischung umfassend ein Dithiol, einen Dialdehyd und einen Katalysator hergestellt wird und wobei das molare Verhältnis von Dithiol zu Dialdehyd mindestens 4:1, vorzugsweise von 6:1 bis 12:1 beträgt.

7. Die Polythiolzusammensetzung gemäß Anspruch 6, wobei das Dithiol das Polythiol gemäß Formel (III), worin n = 2, umfasst; und/oder
wobei der Dialdehyd Glyoxal umfasst; und/oder
wobei der Katalysator Lithiumtetrafluorborat umfasst.

8. Ein Verfahren zum Herstellen der Polythiolzusammensetzung gemäß irgendeinem der vorhergehenden Ansprüche umfassend:
a. Herstellen einer Reaktionsmischung umfassend ein Dithiol, einen Dialdehyd und einen Katalysator, wobei das molare Verhältnis von Dithiol zu Dialdehyd mindestens 4:1 beträgt; und
b. Aussetzen der Reaktionsmischung gegenüber Bedingungen, die ausreichen, um die Polythiolzusammensetzung zu bilden.

9. Das Verfahren gemäß Anspruch 8, wobei das molare Verhältnis von Dithiol zu Dialdehyd mindestens 6:1 beträgt, vorzugsweise von 6:1 bis 12:1.

10. Das Verfahren gemäß irgendeinem der Ansprüche 8 bis 9, wobei das Dithiol ein Polythiol gemäß Formel (III) umfasst 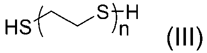 wobei n von 2 bis 4 beträgt.

11. Das Verfahren gemäß irgendeinem der Ansprüche 8 bis 10, wobei der Dialdehyd Glyoxal umfasst; und/oder
wobei der Katalysator Lithiumtetrafluorborat umfasst.

12. Das Verfahren gemäß irgendeinem der Ansprüche 8 bis 11, wobei die Bedingungen, die ausreichen, um die Polythiolzusammensetzung zu bilden, ein Erwärmen auf eine Temperatur von 30 bis 85 °C für 1 bis 24 Stunden umfassen.

13. Eine polymerisierbare Zusammensetzung umfassend:
a) die Polythiolzusammensetzung gemäß irgendeinem der Ansprüche 1 bis 7; und
b) ein aliphatisches Polyisocyanat, wobei das Äquivalentverhältnis von Isocyanatgruppen (-NCO) in dem Polyisocyanat b) zu Thiol (-SH)-Gruppen in der Polythiolzusammensetzung a) von 1:0,9 bis 1:1,1 beträgt.

14. Die polymerisierbare Zusammensetzung gemäß Anspruch 13, wobei das Polyisocyanat b) 4,4'-Methylenbis(cyclohexylisocyanat), Isophorondiisocyanat, eine isomere Mischung von 2,2,4- und 2,4,4-Trimethylhexamethylendiisocyanat und 1,6-Hexamethylendiisocyanat oder Mischungen davon umfasst.

15. Ein optischer Gegenstand gebildet aus der polymerisierbaren Zusammensetzung gemäß irgendeinem der Ansprüche 13 bis 14.

## Revendications

1. Composition de polythiols comprenant :
a) un polythiol conforme à la formule (I) 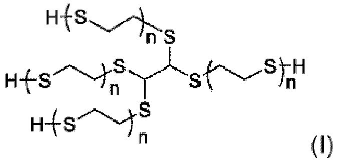 dans laquelle chaque indice n vaut indépendamment de 2 à 4 ; et
b) un polythiol conforme à la formule (II) 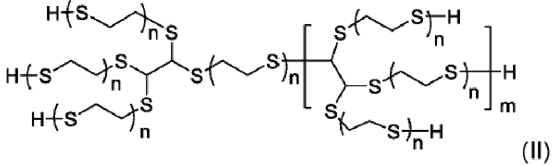 dans laquelle chaque indice n vaut indépendamment de 2 à 4, et m vaut 1 ou 2.

2. Composition de polythiols selon la revendication 1, dans laquelle la composition de polythiols a une masse d'équivalent thiol de 100 à 250 g/équivalent, de préférence de 120 à 160 g/équivalent, par rapport à la masse totale de polythiols présents dans la composition.

3. Composition de polythiols selon l'une quelconque des revendications précédentes, comprenant en outre c) un polythiol conforme à la formule (III) 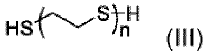 dans laquelle n vaut de 2 à 4.

4. Composition de polythiols selon la revendication 3, dans laquelle n = 2 dans la formule (III).

5. Composition de polythiols selon la revendication 4, dans laquelle le polythiol conforme à la formule (III) est présent dans la composition en une quantité de 10 à 50 pour cent, par rapport aux polythiols totaux dans la composition, comme déterminée par la MÉTHODE LCMS.

6. Composition de polythiols selon l'une quelconque des revendications précédentes, dans laquelle la composition de polythiols est préparée à partir d'un mélange réactionnel comprenant un dithiol, un dialdéhyde, et un catalyseur, et dans laquelle le rapport molaire de dithiol à dialdéhyde est d'au moins 4:1, de préférence de 6:1 à 12:1.

7. Composition de polythiols selon la revendication 6, dans laquelle le dithiol comprend le polythiol conforme à la formule (III) où n est égal à 2 ; et/ou
dans laquelle le dialdéhyde comprend le glyoxal ; et/ou
dans laquelle le catalyseur comprend le tétrafluoroborate de lithium.

8. Procédé de préparation de la composition de polythiols selon l'une quelconque des revendications précédentes, comprenant les étapes consistant à :
a. préparer un mélange réactionnel comprenant un dithiol, un dialdéhyde, et un catalyseur, le rapport molaire de dithiol à dialdéhyde étant d'au moins 4:1 ; et
b. soumettre le mélange réactionnel à des conditions suffisantes pour former la composition de polythiols.

9. Procédé selon la revendication 8, dans lequel le rapport molaire de dithiol à dialdéhyde est d'au moins 6:1, de préférence de 6:1 à 12:1.

10. Procédé selon l'une quelconque des revendications 8 et 9, dans lequel le dithiol comprend
un polythiol conforme à la formule (III) 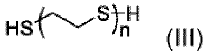
dans laquelle n vaut de 2 à 4.

11. Procédé selon l'une quelconque des revendications 8 à 10, dans lequel le dialdéhyde comprend le glyoxal ; et/ou
dans lequel le catalyseur comprend le tétrafluoroborate de lithium.

12. Procédé selon l'une quelconque des revendications 8 à 11, dans lequel les conditions suffisantes pour former la composition de polythiols comprennent un chauffage jusqu'à une température de 30 à 85 °C pendant 1 à 24 heures.

13. Composition polymérisable comprenant
a) la composition de polythiols selon l'une quelconque des revendications 1 à 7 ; et
b) un polyisocyanate aliphatique, le rapport d'équivalents des groupes isocyanate (-NCO) dans le polyisocyanate b) aux groupes thiol (-SH) dans la composition de polythiols a) valant de 1:0,9 à 1:1,1.

14. Composition polymérisable selon la revendication 13, dans laquelle le polyisocyanate b) comprend le 4,4'-méthylène-bis(cyclohexylisocyanate), le diisocyanate d'isophorone, un mélange d'isomères de diisocyanate de 2,2,4- et 2,4,4-triméthylhexaméthylène, et le 1,6-diisocyanate d'hexaméthylène, ou des mélanges de ceux-ci.

15. Article optique formé à partir de la composition polymérisable selon l'une quelconque des revendications 13 et 14.
